# EUROPEAN PATENT SPECIFICATION

(11) **EP 4 274 570 B1**
(45) Date of publication and mention of the grant of the patent: **24.12.2025**
(21) Application number: 22704423.7
(22) Date of filing: 11.01.2022
(51) Int. Cl.: A61K 31/436, A61K 45/06, A61P 21/00, C12N 15/113, A61K 31/713

(54) **ACTIVATORS OF THE BMP/SMAD AXIS FOR USE IN TREATING AND/OR PREVENTING MUSCLE ATROPHY**
AKTIVATOREN DER BMP/SMAD-ACHSE ZUR VERWENDUNG BEI DER BEHANDLUNG UND/ODER VORBEUGUNG VON MUSKELSCHWUND
ACTIVATEURS DE L'AXE BMP/SMAD POUR UTILISATION DANS LE TRAITEMENT ET/OU LA PRÉVENTION DE L'ATROPHIE MUSCULAIRE

(30) Priority: 11.01.2021 IT 202100000368
(43) Date of publication of application: 15.11.2023
(73) Proprietor: Università Degli Studi Di Torino, 10124 Torino (IT); Fondazione Centro San Raffaele, 20132 Milano (IT)
(72) Inventor: PORPORATO, Paolo Ettore, Torino (TO) (IT); MINA, Erica, Torino (TO) (IT); RAUSCH, Valentina, Torino (TO) (IT); SILVESTRI, Laura, Milano (MI) (IT); MAZZONE, Massimiliano, Torino (TO) (IT)
(74) Representative: Pace Napoleone, Maria
(86) International application number: PCT/IB2022/050175
(87) International publication number: WO 2022/149113

(56) References cited:
- EP-A1- 2 705 856
- WO-A1-2018/185341
- US-A1- 2002 123 456
- US-B2- 9 730 967
- DAVID W HAMMERS ET AL: "Supraphysiological levels of GDF11 induce striated muscle atrophy", EMBO MOLECULAR MEDICINE, vol. 9, no. 4, 7 March 2017 (2017-03-07), US, pages 531 - 544, XP055718947, ISSN: 1757-4676, DOI: 10.15252/emmm.201607231
- SARTORI ROBERTA ET AL: "BMP signaling controls muscle mass", vol. 45, no. 11, 1 November 2013 (2013-11-01), pages 1309 - 1318, XP009510379, ISSN: 1061-4036, Retrieved from the Internet <URL:http://www.nature.com/articles/ng.2772> [retrieved on 20130929], DOI: 10.1038/NG.2772
- KEISUKE HITACHI ET AL: "Long Non-Coding RNA Myoparr Regulates GDF5 Expression in Denervated Mouse Skeletal Muscle", NON-CODING RNA, vol. 5, no. 2, 8 April 2019 (2019-04-08), pages 33, XP055672975, DOI: 10.3390/ncrna5020033
- CRAIG A GOODMAN ET AL: "New roles for Smad signaling and phosphatidic acid in the regulation of skeletal muscle mass", F1000PRIME REPORTS, vol. 6, 1 January 2014 (2014-01-01), England, pages 20, XP055297579, DOI: 10.12703/P6-20
- GRECO STEPHANIE H. ET AL: "TGF-[beta] Blockade Reduces Mortality and Metabolic Changes in a Validated Murine Model of Pancreatic Cancer Cachexia", vol. 10, no. 7, 14 July 2015 (2015-07-14), pages e0132786, XP055840560, Retrieved from the Internet <URL:https://journals.plos.org/plosone/article?id=10.1371/journal.pone.0132786> DOI: 10.1371/journal.pone.0132786
- ZHENG CHENLEI ET AL: "Exploring the Mechanism of Skeletal Muscle in a Tacrolimus-Induced Posttransplantation Diabetes Mellitus Model on Gene Expression Profiles", vol. 2020, 10 January 2020 (2020-01-10), pages 1 - 11, XP055840174, ISSN: 2314-6745, Retrieved from the Internet <URL:https://downloads.hindawi.com/journals/jdr/2020/6542346.pdf> DOI: 10.1155/2020/6542346
- FEI SUN ET AL: "Design and Structure-Based Study of New Potential FKBP12 Inhibitors", BIOPHYSICAL JOURNAL, vol. 85, no. 5, 1 November 2003 (2003-11-01), AMSTERDAM, NL, pages 3194 - 3201, XP055543385, ISSN: 0006-3495, DOI: 10.1016/S0006-3495(03)74737-7
- EDDA SPIEKERKOETTER ET AL: "FK506 activates BMPR2, rescues endothelial dysfunction, and reverses pulmonary hypertension", JOURNAL OF CLINICAL INVESTIGATION, vol. 123, no. 8, 15 July 2013 (2013-07-15), pages 3600 - 3613, XP055140950, ISSN: 0021-9738, DOI: 10.1172/JCI65592

## Description

### FIELD OF THE INVENTION

The present invention relates to an activator of the BMP-SMAD signaling pathway for use in the treatment and/or prevention of muscle atrophy or for use in a therapeutic method for the promotion and/or induction of muscle hypertrophy and relative pharmaceutical composition for said use, as defined in the claims.

### BACKGROUND ART

Tumor cachexia is a disabling syndrome found in 80% of different types of tumors and is not only related to the worsening of the prognosis and response to anticancer therapies, but is also considered the cause of death due to heart or respiratory failure of at least 20% of cancer patients. Despite the high prevalence of the syndrome, there are currently no specific treatments which counteract the onset and development of the disease (1, 2).

From a biological point of view, neoplastic cachexia is mainly characterized by systemic inflammation and a decrease of at least 5% of body weight, given by the loss of adipose tissue and a strong atrophy in skeletal muscles (1).

In particular, muscular atrophy is caused by an imbalance in protein synthesis and degradation, as a result of the activation of TGF_{β} signal pathways. The TGF_{β} receptor superfamily is physiologically involved in the regulation of muscle mass. Inflammatory molecules such as Activin A (ACTA) or TGF_{β}, abundant in systemic inflammation conditions such as cachexia, bind and activate TGF_{β} receptors (ActRIIB, ActRIIA, TGF_{β}RII and ALK-4, -7, -5), inducing the phosphorylation of the SMAD2/3 proteins and the expression of E3 ubiquitin ligase (atrogin 1, MURF-1 and MUSA1), known as "atrogens", which represent a signal of protein degradation and therefore, of muscle atrophy.

Conversely, ligands of the BMP family, binding specific BMP receptors (BMPRII, ActRIIB, ActRIIA, and ALK-2, -3), induce a protein biosynthesis signal through the phosphorylation of the SMAD1/5/8 proteins and activation of the Akt/mTOR axis. (3, 22). Compound LDN-193189 is a non-specific inhibitor of the kinase activity of receptors belonging to the TGF_{β} superfamily and inhibits both the BMP and TGF_{β} receptors (ALK1, ALK2, ALK3, ALK4, ALK5, ALK6). Mice treated for 9 days with LDN-193189 showed a reduction in baseline phosphorylation levels of Smad1/5/8 and 21% atrophy in the myofibers of the tibialis anterior.

Furthermore, the inhibition of BMP receptor-mediated signaling pathways and the knockdown of Smad1/5 has been shown to be sufficient to inhibit Smad1/5/8 phosphorylation and induce muscle atrophy. On the other hand, the overexpression of BMP7 and caALK3 were sufficient to increase Smad1/5/8 phosphorylation and induce substantial muscle hypertrophy (23). Recently in a hepatocyte culture model, the immunophylline FKBP12 (FK506-binding protein) has been shown to interact with the BMP ALK2 receptor to avoid the excessive activation of the BMP-SMAD1/5/8 signaling pathway. In this study, the drug tacrolimus (FK506) was shown to bind FKBP12, causing it to detach from the ALK2 receptor, thereby increasing the phosphorylation of SMAD 1/5/8 proteins and the transcription of their epcidin target into hepatocytes.(4) At the muscle level, there is no information on the possible role of FK506 and on the interaction between FKBP12 and the BMP receptors.

The drug tacrolimus is currently in use in the clinic as an immunosuppressant due to the promiscuity of the target protein, i.e., when it is in multimeric complex with calcineurin and FKBP12 (5).

The immunosuppressive activity of tacrolimus is strictly dose-dependent: high immunosuppressive doses, corresponding to a blood concentration between 4 and 20 ng/ml, induce calcineurin inhibition and, therefore, the activation of T lymphocytes, whereas low non-immunosuppressive doses only interfere with the interaction between FKBP12 and the BMP receptor, as previously demonstrated in the hepatocyte (4) and renal (14) context. In an animal model of post-transplant diabetes mellitus rats, used at high immunosuppressive doses (1 mg/kg/d), tacrolimus induces muscle atrophy (24).

US-B2-9730967 discloses a composition comprising bacillus calmette-guerin adapted to produce tomatidine and rapamycin for use in treating cancer cachexia.

Therefore, there is still a need for specific treatments which counteract the onset and development of muscle atrophy. In the course of the present invention it has been surprisingly found that doses of tacrolimus at least 30 times lower with respect to the doses used in post-transplant treatments to obtain immunosuppression, activate the BMP-SMAD1/5/8 signaling pathway at the muscular level allowing to counteract processes of muscular atrophy.

### SUMMARY OF THE INVENTION

The references to methods of treatment in this description are to be interpreted as references to the compounds, pharmaceutical compositions and medicaments of the present invention for use in a method for treatment of the human (or animal) body by therapy.

The present inventors have surprisingly found that the modulation of the biosynthetic BMP-SMAD axis by interaction with FKBP12 allows to counteract the process of muscular atrophy, in particular associated with a reduced BMP-SMAD axis regardless of a tumor context (e.g., prolonged immobility or bed rest or immobilization syndrome (6)) or to counter the process of muscular atrophy induced by tumor cachexia.

In the course of the present invention it has been surprisingly observed that modulators of biosynthetic BMP-SMAD axis capable of intercepting FKBP12 immunophyllin regulate muscle mass, specifically targeting the molecular mechanism of muscle atrophy, in particular in tumor cachexia. Such biosynthetic modulators of biosynthetic BMP-SMAD axis capable of intercepting immunophylline FKBP12 comprise in particular tacrolimus, also known as FK-506, and its structural analogues. According to the invention, tacrolimus and its analogues as defined in the claims counteract muscular atrophy by administration at non-immunosuppressive doses. Importantly, these effects are specific for non-immunosuppressive doses, while immunosuppressive dosages are shown to promote atrophy (24).

It has been unexpectedly observed that modulators of biosynthetic BMP-SMAD axis are able to increase SMAD1/5 phosphorylation levels, without the stimulation of any extracellular ligand. Furthermore, they completely reverse the significant increase in protein ubiquitination induced by the TGF_{β} receptor ligand, Activin A.

In the present disclosure it is demonstrated that the biological effect of the modulators of biosynthetic BMP-SMAD axis on myotubes, in particular tacrolimus, is independent of calcineurin inhibition. Furthermore, they are capable of preventing muscle atrophy, both in terms of skeletal muscle weight and in terms of muscle fiber diameter. As a further demonstration of atrophy protection, such modulators reduce the expression of all the atrogens in the gastrocnemes of C26 mice treated with the modulator, which represent the marker genes of the atrophic muscles.

The treatment with modulators of biosynthetic BMP-SMAD axis is not only capable of preventing conditioned medium-induced atrophy of colon-26 (C26 CM) tumor cells in C2C12 myotubes, but is also capable of inducing significant hypertrophy. In particular, the modulators are activators of the biosynthetic axis of BMP-SMAD.

In the present disclosure the enhancement or activation of the BMP-SMAD axis is achieved in particular with the removal of FKBP12, more specifically with the displacement of FKBP12 from the ALK2 BMP receptor. In the case of tacrolimus, the BMP-SMAD signaling pathway activator does not have immunosuppressive activity, as the doses used are not sufficient to form the multimeric complex with calcineurin, but only concern the interaction with the FKBP12 protein.

In the present disclosure it has been surprisingly observed that the protective effects on the muscle are also present following removal of the FKBP12 protein, for example with non-coding RNAs such as siRNAs or shRNAs. Therefore, the present disclosure further relates to the use of vectors for targeting interfering RNA or shRNA for *FKBP12* or to the use of peptides for displacing FKBP12 interaction with receptors involved in BMP-SMAD signaling. The novelty of this approach lies in limiting the promiscuity of the FKBP12 protein by reducing the expression thereof by means of interfering RNAs without inhibiting the activity thereof. This mechanism avoids the non-specific effects which are observed following pharmacological inhibition of the protein (e.g., inhibition of mTOR and calcineurin).

Since the treatment of neoplastic cachexia is not a routine clinical treatment due to the lack of drugs which directly affect the molecular process, the use of modulators of the biosynthetic axis of BMP-SMAD could contribute to the sector of this syndrome, avoiding the long and expensive processes of development of a new drug.

Therefore, the present invention provides an activator of the BMP-SMAD signaling pathway for use in the treatment and/or prevention of muscle atrophy, in which said activator is an FKBP12 inhibitor not inhibiting mTOR selected from the group consisting of tacrolimus or an analogue thereof selected from FKVP, L-658,818 or V-10,367 or GPI-1046 or GPI-1485, FK523 or FK520, or 36,37-dihydro-FK506, used at doses which do not induce immunosuppression, siRNA targeting FKBP 12, and shRNA targeting FKBP 12.

Preferably the FKBP12 inhibitor displaces FKBP12 from a BMP receptor, preferably said receptor is ALK1, ALK2 or ALK3.

According to the present disclosure, an FKBP12 inhibitor is a molecule capable of interacting with FKBP12 by preventing the binding thereof to a BMP receptor or a molecule which inhibits the expression or function of FKBP12.

Preferably, according to the disclosure, the FKBP12 inhibitor which activates the BMP-SMAD signaling pathway is selected from the group consisting of: a small molecule, a peptide, a protein, an antisense oligonucleotide, a siRNA, a shRNA, a recombinant expression vector or virus, an antibody or a fragment thereof.

Preferably said inhibitor is a non-coding RNA, such as a siRNA or an analogue such as shRNA, interfering RNA or gRNA, preferably a siRNA. According to an embodiment of the invention, the *FKBP12* target siRNA.

Preferably said inhibitor is a shRNA. According to an embodiment of the invention, said *FKBP12* target shRNA.

According to an embodiment of the invention, said inhibitor is tacrolimus or a structural analogue thereof, as defined in the claims.

Preferably said inhibitor is used daily orally at non-immunosuppressive doses, therefore not altering the calcineurin activation state. Immunosuppressive doses of tacrolimus are doses capable of providing a blood concentration of the drug in vivo between about 4 and about 20 ng/ml and are obtained by oral administration at doses between about 0.1 mg/kg/day and about 1 mg/kg/day.

A further object of the invention is tacrolimus for use in the treatment and/or prevention of muscle atrophy, wherein preferably the tacrolimus is used at non-immunosuppressive doses.

The present disclosure provides a method for treating and/or preventing muscle atrophy in a patient in need thereof by administering an FKBP12 inhibitor which activates the BMP-SMAD signaling pathway, as defined herein.

Preferably the muscle atrophy is selected from the group consisting of: neoplastic cachexia, bed rest or immobilization syndrome, atrophy associated with heart and/or kidney failure condition, atrophy associated with systemic inflammatory response syndrome (Sirs), preferably the systemic inflammatory response syndrome is AIDS, COPD, sepsis or hepatic steatosis.

Preferably the muscle atrophy is characterized by a reduced BMP-SMAD axis. Reduced BMP-SMAD axis means a reduction in the phosphorylation of at least one of the SMAD1, SMADS or SMAD8 proteins, which are those that are phosphorylated when BMP ligands bind and activate the BMP receptors. This occurs in different models of pathologic muscle atrophy such as denervation and cachexia (7) and/or by an increase in the ACTA/TGF_{β}-SMAD2/3 signal pathway when compared to non-atrophic muscles (7). An increase in the TGF_{β}/ACTA-SMAD axis means an increase in the phosphorylation of at least one of the SMAD2 and/or SMAD3 proteins, which are those which are phosphorylated when ligands such as TGF_{β} and/or Activins bind to and activate the TGF_{β} receptors. It is measured by evaluating the phosphorylation intensity with immunoblotting and/or luciferase assays (CAGA-Luc reporters) and/or transcriptional targets regulated by the SMAD2/3 axis as listed in (7).

Preferably the FKBP12 inhibitor is used in combination with a therapeutic agent and/or a therapeutic intervention.

Preferably the therapeutic agent and/or intervention is selected from the group consisting of TGF_{β} signal pathway antagonists e.g., monoclonal antibodies such as fresolimumab, galunisertib and many other molecules (e.g., SM16, YR-290 etc.), or agonists (e.g., SB4 (25)) or activators (e.g., tilorone (26)) of the BMP signal pathway.

Preferably, the therapeutic agent and / or the intervention is selected from the group consisting of: chemotherapeutics belonging to different categories, such as DNA alkylators (e.g., cyclophosphamide), monoclonal antibodies which target specific mutated proteins, antimetabolites (e.g., fluorouracil, methotrexate), cell proliferation inhibitor drugs (e.g., taxanes), orexants (peripheral orexants such as strychnine and orexin; or central orexants such as monoamine oxidase inhibitors, e.g., isoniazide), some tricyclic antidepressants (e.g., amitriptyline), TGF_{β} signal pathway antagonists e.g., monoclonal antibodies such as fresolimumab, galunisertib and many other molecules (e.g., SM16, YR-290, etc.) and immunostimulants (e.g., imiquimod, resiquimod and vaccines) including checkpoint inhibitors (monoclonal anti-CTLA4 antibodies (e.g., ipilimumab), anti-PD1 (e.g., nivolumab) and anti-PDL1 (e.g., atezolizumab)) and CAR-T.

The present invention also provides an activator of the BMP-SMAD signaling pathway for use in a therapeutic method for the promotion and/or induction of muscle hypertrophy, in which said activator is a FKBP12 inhibitor, as defined in the claims.

The present disclosure also provides the use of an activator of the BMP-SMAD signaling pathway, in which said activator is an FKBP12 inhibitor, in the preparation of a medicine for the promotion and/or induction of muscle hypertrophy.

The present invention provides a pharmaceutical composition comprising an activator of the BMP-SMAD signaling pathway as defined in the claims for use in the treatment and/or prevention of muscle atrophy, or for use in a therapeutic method for the promotion and/or induction of muscle hypertrophy, preferably said composition further comprising a therapeutic agent, preferably as defined above.

An activator of the BMP-SMAD signaling pathway which inhibits FKBP12 increases the phosphorylation levels of at least one of the SMAD1, SMAD5, or SMAD8 proteins, and induces anabolic signaling such as AKT-mTOR and protein synthesis.

In particular, said activator causes the detachment of FKBP12 from the BMP receptors allowing these receptors to work more effectively in the activation of the BMP/SMAD pathway.

The dislocation of the protein from the receptor is measured by methods known in the art, e.g., expressing BMP-tagged receptors and performing a Pull-down assay of the receptor, displaying the absence or presence of the FKBP12 protein with immunoblotting, with and without activator.

Preferably the activator is a molecule which is a ligand of proteins involved in the BMP-SMAD axis.

The activation or reduction/inhibition of the BMP-SMAD signaling pathway can be measured through Immunoblotting of phosphorylations (phospho-Serine 463/465-SMAD1/5/8), through the quantification of the luminescence signal of the BRE-Luc reporter vector and through the quantification of the expression levels of BMP-SMAD target genes (e.g., *ID1, ID3* for hepatocytes) or any other method known in the art.

According to the invention, tacrolimus analogues are non-immunosuppressive structural analogues and incapable of inhibiting mTOR. Structural analogs of tacrolimus according to the invention are selected from synthetic compounds selected from FKVP (manufactured by Peiffer, CellPress 2019 - (8)), L-658,818 or V-10,367 or GPI-1046 or GPI-1485 or tacrolimus derivatives with modified C21 side chains, selected from FK523 or FK520, or 36,37-dihydro-FK506.

Tacrolimus analogues according to the invention are preferably capable of displacing the FKBP12 protein from the BMP, ALK1, ALK2 or ALK3 receptor, preventing the binding thereto and capable of activating Smad1/5/8 phosphorylation when used at doses which do not activate further signaling pathways or have immunosuppressive effect.

The invention will be illustrated by the following non-limiting figures and examples.

For all the figures, the statistics are as follows: The error bars indicate SEM. *P <0.05; **P<0.01; ***P<0.001; ****P<0.0001. The asterisk indicates the statistical significance towards a sample group other than the control.

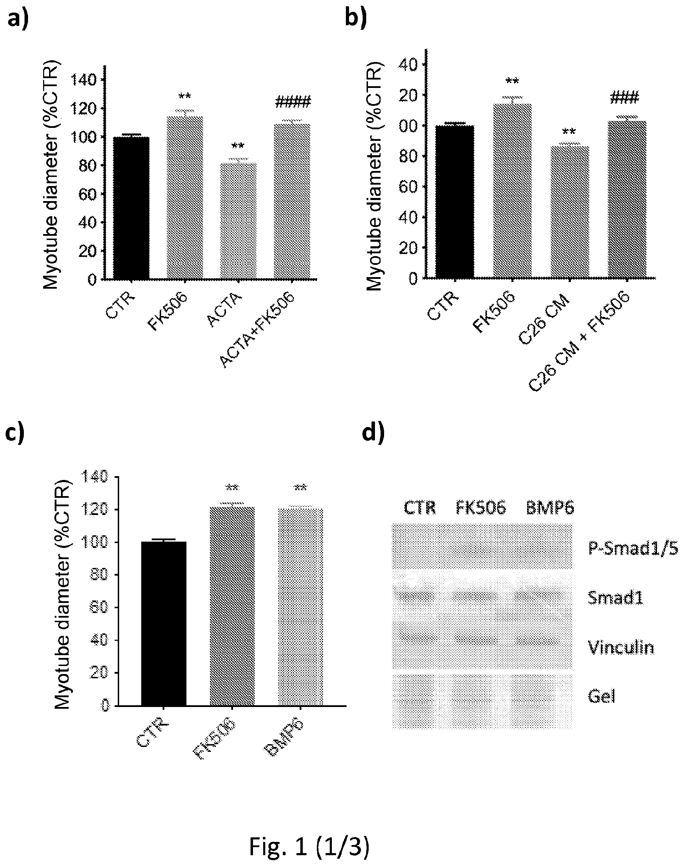

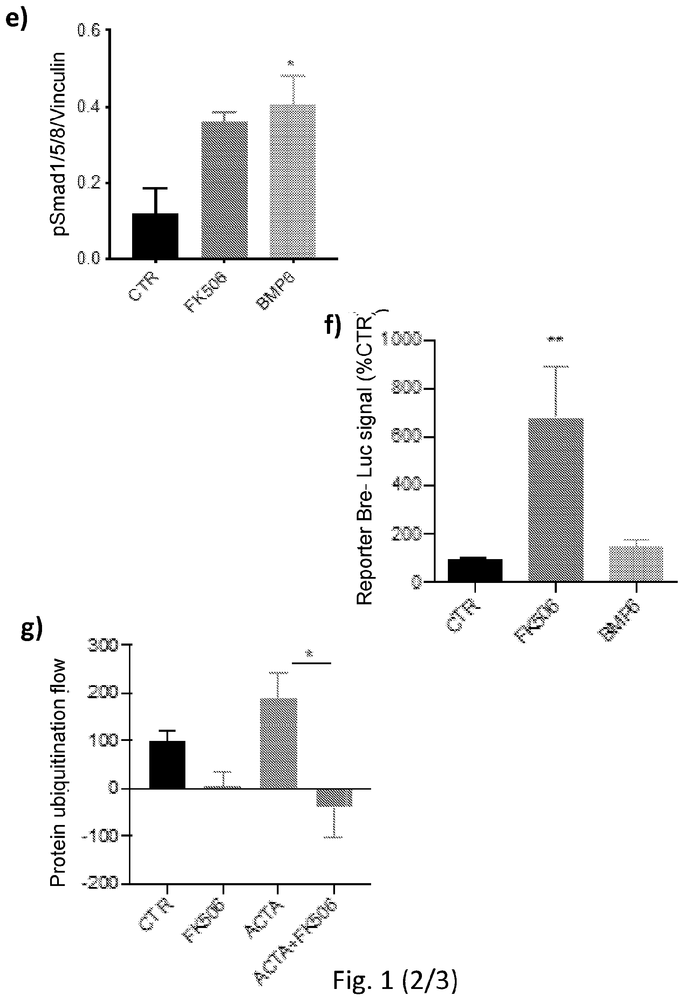

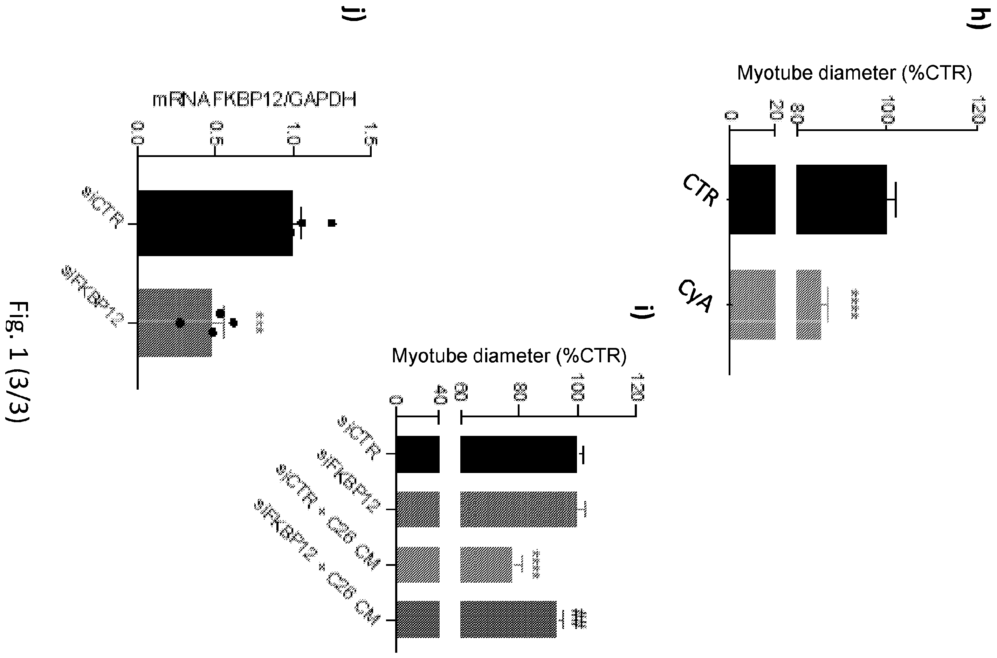

**Fig. 1 a-c****)** Diameters of C2C12 myotubes after 24h of treatment with FK506 1µg/mL, Activin A 1ng/mL (ACTA), conditioned medium C26 or BMP6 1ng/mL or vehicle (CTR) (n=3-4). **d-e)** Western blot representative of SMAD1/5 phosphorylation in C2C12-myotubes after 1h of treatment with FK506 1µg/mL or BMP6 30ng/mL (d) and relative quantification (s) (n=3). **f)** Intensity of the luminescence signal in C2C12 myoblasts, transfected with the plasmid pGL3-BMP responsive element (BRE)-Luc and treated for 5h with FK506 1µg/mL or BMP6 1ng/mL (n=3). **g)** Measurement of protein ubiquitination flow in C2C12 myotubes treated with FK506 1µg/mL, Activin A 1ng/mL (ACTA), and the combination of the two, with and without MG132 1µM for 1h (n=2-3). **h)** Diameters of C2C12 myotubes after 24h of treatment with cyclosporine A 1µM (CyA) (n=3). **i)** Diameters of C2C12 myotubes transfected with siFKBP12 and treated with C26 CM for 24h (n=3). **j)** mRNA levels of FKBP12 in C2C12 myotubes transfected with and treated with C26 CM for 24h (n=3).

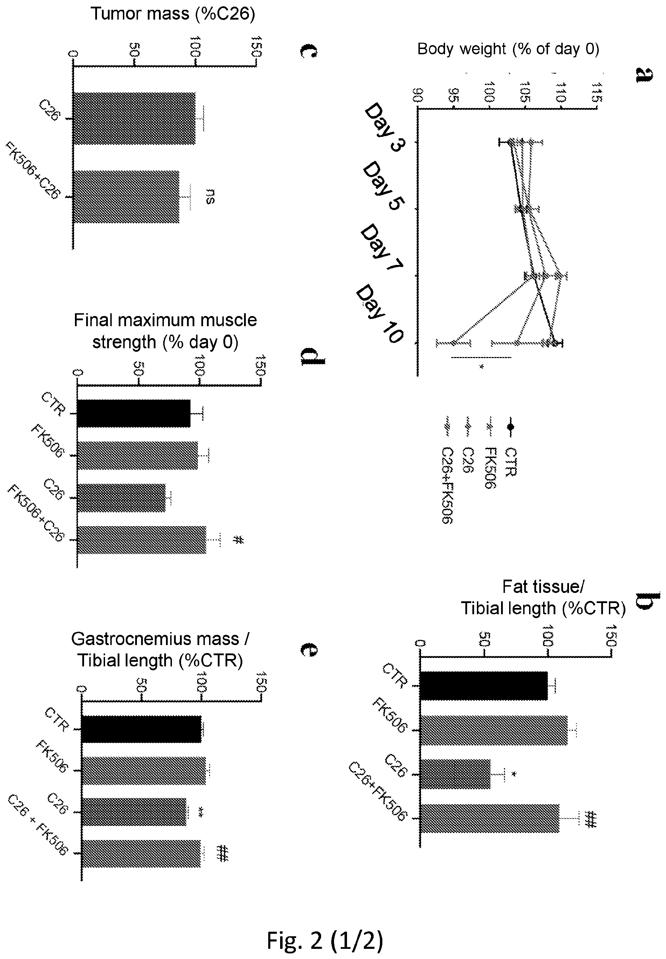

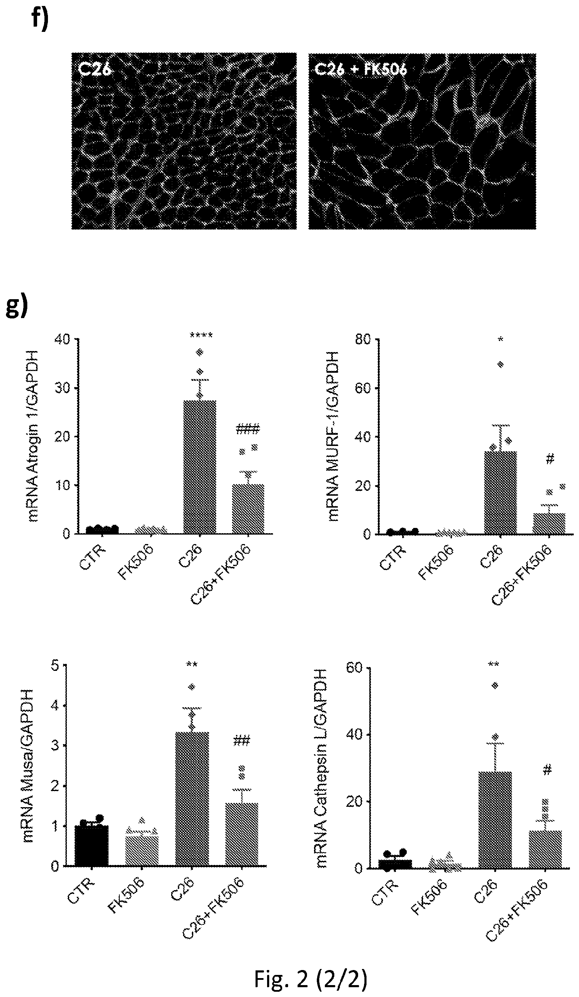

**Fig. 2 a****)** Body weight trend of control (CTR) or C26 mice treated orally with FK506 or vehicle. (n=5-7). **b)** Weight of the subcutaneous and inguinal adipose tissue normalized for the length of the tibia (n=6-7). **c)** Weight of the tumor mass at the time of sacrifice (n=12). **d)** Maximum muscle strength before sacrifice in percentage with respect to the beginning of the experiment. **e)** Final weight of the gastrocnemius normalized for the length of the tibia. **f)** Representative image of an immunofluorescence for laminin (green) and DAPI (blue) of a gastrocnemius cross-section. **g)** Atrogen mRNA levels (Atrogin 1, MuRF-1, Musa1, Cathepsin L) normalized for GAPDH levels in the gastrocnemii. (n=4-6).

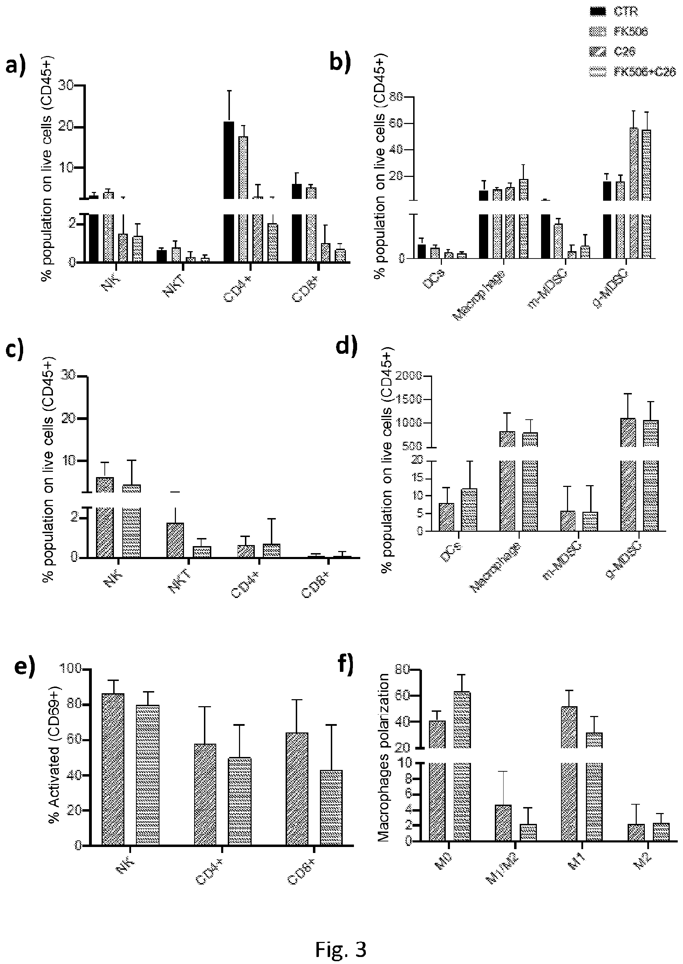

**Fig. 3 a****)** Percentage of lymphocyte populations in blood. (n=4-6) **b)** Percentage of myeloid populations in blood (m-MDSC: monocytic myeloid-derived suppressor cells; g-MDSC: granulocytic myeloid-derived suppressor cells) (n=4-6) **c)** Percentage of lymphocyte populations in C26 tumors. (n=6) **d)** Percentage of myeloid populations in C26 tumors. (n=6) **e)** Percentage of activated lymphocyte populations (CD69+) in C26 tumors. (n=6) **f)** Polarization status of macrophages in C26 tumors. (n=6)

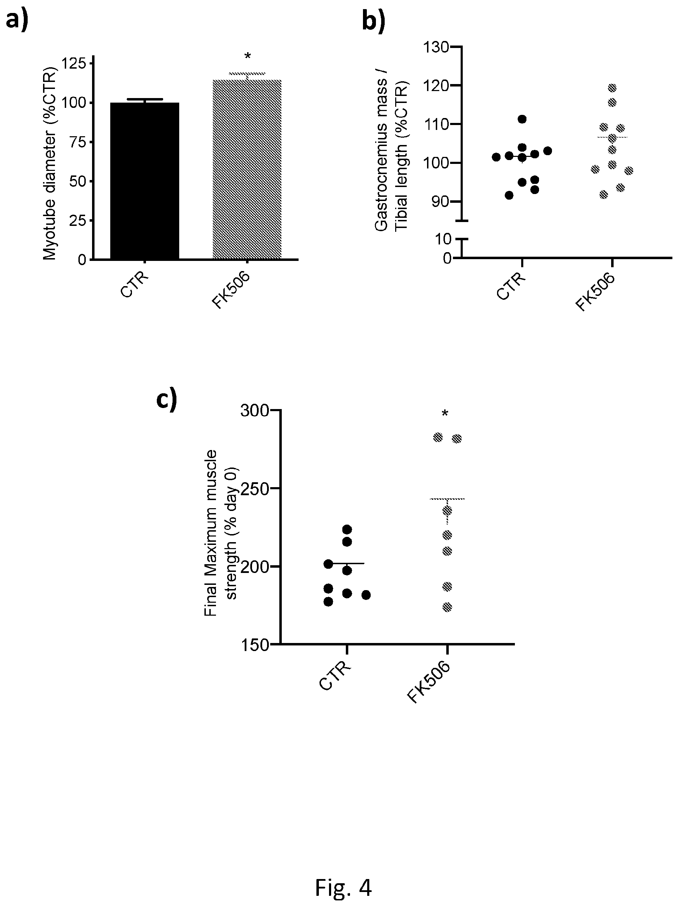

**Fig. 4 a****)** Diameters of C2C12 myotubes after 24h of treatment with FK506 1µg/mL. **b)** Final weight of gastrocnemius normalized for tibia length, after 11 days of oral treatment with FK506. **c)** Maximum muscle strength after 4 weeks of oral treatment with FK506.

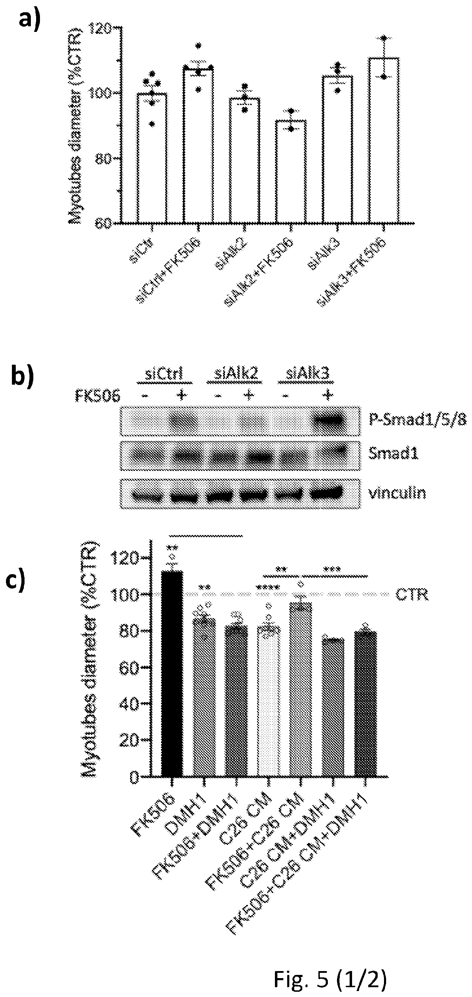

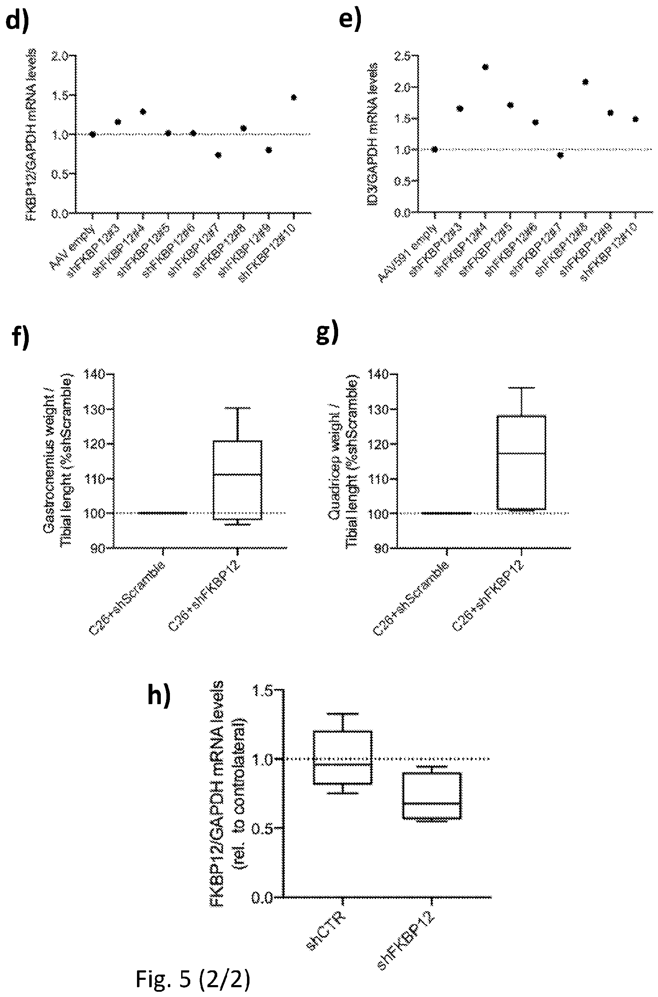

**Fig. 5 a****)** Diameters of C2C12 myotubes transfected with siRNA for the BMP ALK2 and ALK3 receptors, with and without tacrolimus (FK506 1µg/mL). **b)** Immunoblot showing the phosphorylation levels of the Smad1/5/8 samples in Fig. 1a. **c)** Diameters of C2C12 myotubes following 24h of treatment with DMH1 (5uM), tacrolimus (FK506 1µg/mL) and C26 conditioned medium (10%). **d)** Evaluation of FKBP12 silencing with different AAV CRE:shRNA constructs in C2C12 myotubes. e) ID3 expression levels of the samples in Fig. 1d. **f)** Weight of gastrocnemii and quadriceps **(g)** infected with AAV9-shScramble or AAV9-shFKBP12, normalized for tibia length at the end of the C26 cachexia experiment. **h)** FKBP12 expression levels in gastrocnemii at the end of the C26 experiment (day 11) infected with AAV9-shScramble or AAV9-shFKBP12.

### EXAMPLES

### MATERIALS AND METHODS

### Cell cultures

C2C12 myoblasts (ATCC CRL-1772) are proliferated in DMEM/10% FBS medium. When the confluence of 80-90% is reached, they are differentiated into myotubes in DMEM/2% HS medium for 4 days.

The myotubes were treated with 10% C26 conditioned medium (C26 CM (9)) or Activin A (1ng/mL R&D # 338-AC), FK506 (1µg/mL Cayman Chemical, #10007965), BMP6 (1ng/mL R&D Systems, 6325-BM), cyclosporine A (1µM Tocris Biosciences #1101), DMH1 (5µM CAS No 1206711-16-1) or with DMEM/2%HS (CTR) differentiation medium for 24h to measure their diameter. For the signaling experiments, two hours prior to treatment, the DMEM differentiation medium without serum was changed, followed by 1h of treatment with FK506 (1 µg/mL) or BMP6 (30ng/mL). The protein ubiquitination flow was measured by treating in C2C12 myotubes with FK506 1µg/mL, Activin A 1ng/mL (ACTA), and the combination of the two, with and without MG132 proteasome inhibitor (Senta Cruz Biotechnology, SC-351846), 1µM for 1h.

### Transfection

For RNA interference experiments, C2C12 myoblasts are differentiated for 3 days before being transfected with siFKBP12 (QIAGEN, SI00171052, Gene ID 14225), siALK2 (QIAGEN, S100888874), siALK3 (QIAGEN, #SI00929971) using Lipofectamine RNAiMax (Thermo Fisher, #13778075). The myotubes were analyzed 48h after transfection to establish the diameters and lysates for RNA extraction.

### Luciferase assay

C2C12 myoblasts were transfected with the pGL3-BMP responsive element (BRE)-Luc plasmid (Addgene #45126) to measure the promoter activation by the SMAD1/5/8 proteins. The cells were treated with FK506 (1µg/mL) or BMP6 (1ng/mL) 15h posttransfection. The luminescence was measured using Dual-Luciferase Reporter Assay System (Promega) after 5h of treatment according to the manufacturer's instructions.

### Measurement of the myotube diameters.

Photographs of the myotubes were taken using 20X magnification bright field microscopy (Zeiss) and their diameter was measured using FIJI software.

### Western Blotting

C2C12 myotubes were lysed in RIPA lysis buffer (150mM NaCl, 50mM Tris-HCl, 0.5% sodium deoxycholate, 1.0% Triton X-100, 0.1% SDS, and 1mM EDTA) with protease and phosphatase inhibitors (Roche, #0490837001, #11697498001). The protein concentration was determined using the BCA assay (Thermo Fisher Scientific). 15µg of proteins from cell lysates were loaded for performing SDS-PAGE, then transferred onto PVDF membrane for immunoblotting analysis. The primary antibodies used are: SMAD1 (Cell Signaling, #9743), P-SMAD1/5 (Cell Signaling, #13820T), vinculin (Cell Signaling #4650), ubiquitin (Abcam, #7780).

### In vivo experiments

All the experiments with animals were authorized by the Ministry of Health of the Italian Republic in accordance with the European Community guidelines for the use and well-being of animals.

FK506 (Cayman Chemical, #10007965) was dissolved in DMSO (5mg/mL) and then diluted in a 5% glucose and water solution for oral administration in order to reach the final concentration of 0.02mg/kg. All the mice were subjected to one day of treatment, prior to inoculation of colon-26 (C26) adenocarcinoma cells into the right flank of three-month-old Balb C females (Charles River #028) (0.7x106 cells/mouse). All the mice were treated daily with FK506 (0.02 mg/Kg) or 5% glucose buffer DMSO vehicle.

Maximum muscle strength was measured on the day of inoculation (day 0) and the day of sacrifice (day 11) using the Grip Strength Test (Bioseb).

The gastrocnemii, tumors, and subcutaneous and inguinal adipose tissue were isolated, weighed, and normalized for the tibia length of each mouse.

### Hypertrophy experiment (Fig. 4d)

4-month-old Balb C females were treated orally every day for 4 weeks (5 days per week), with 0.02mg/Kg/day FK506 in 5% glucose solution or with vehicle (CTR). The maximum strength was measured weekly with the Grip Strength Test.

### Histology

The fresh gastrocnemii were frozen in liquid nitrogen-cooled isopentane and stored at -80°C. 7µm cross-sections were performed using a cryostat. Immunofluorescence was performed by securing the tissues in 4% PFA, blocking with 0.1%, 3% BSA in PBS before incubating with the primary antibody for laminin (sc-59854), and with the secondaries (ALEXA488) and DAPI (Abcam 228549). The photographs were taken with a fluorescence microscope to evaluate the cross-sectional area of the fibers.

### RNA extraction and Real Time PCR

The total RNA from the frozen tissues was extracted with TRIzol reagent (Invitrogen) following the manufacturer's instructions. 0.5-1µg of total RNA were retrotranscribed using High Capacity cDNA Reverse Transcriptase kit (Applied Biosystems). The cDNA was analyzed with Real Time Quantitative PCR (ABI PRISM 7900HT FAST Real-Time PCR system, Applied Biosystems) using Luna Universal Probe qPCR master mix (Nebb) and Universal Probe Library system (Roche Applied Science). The relative mRNA levels were calculated using the 2-ΔΔCT method and normalizing for the GAPDH levels. The following primers were used: Atrogin1 F: 5'-agtgaggaccggctactgtg-3' (SEQ ID No. 1), R: 5' gatcaaacgcttgcgaatct-3' (SEQ ID No. 2); Murf1 F: 5'tgacatctacaagcaggagtgc-3' (SEQ ID No. 3), R: 5'-tcgtcttcgtgttccttgc-3' (SEQ ID No. 4); Musa1 F: 5'-gagaagccagggtttgagc-3' (SEQ ID No. 5), R: 5'-tcatacagtgtgagtgctgctg-3' (SEQ ID No. 6); Cathepsin L F: 5'-gtggactgttctcacgctcaag-3' (SEQ ID No. 7), R: 5'-tccgtccttcgcttcatagg-3' (SEQ ID No. 8); FKBP12 F: 5'-atgggagtgcaggtggag-3' (SEQ ID No. 9), R: 5'-tctttccatcttcaagcatcc-3' (SEQ ID No. 10). GAPDH F: 5'-gtgaaggtcggtgtgaacgg-3' (SEQ ID No. 11), R: 5'-gtgaaggtcggtgtgaacgg-3' (SEQ ID No. 12).

### Facs

The blood was extracted and incubated with erythrocyte lysis buffer for 10 minutes at room temperature. After washing with PBS, the cells were collected, incubated with Fc-receptor blocker (anti-CD16/CD32 antibody, BD Bioscience #101302) and stained for 30 minutes at 4°C with the antibodies.

The primary tumors were extracted, crushed, and digested with collagenase IV (1mg/mL) (Sigma Aldrich #C4-28) in RPMI-1640 at 37°C for 1h. After washing the tissues with RPMI-1640/10% FBS, the suspension was passed through a 70-µm pore filter, centrifuged at 1400rpm for 10 minutes and incubated with the erythrocyte lysis buffer (155 mM NH4Cl, 15.8 mM Na2CO3, 1 mM EDTA, pH 7.3) for 10 minutes at room temperature. After washing with RPMI-1640/10% FBS, 1 million cells were resuspended in PBS, treated with Fc-receptor blocker (anti-CD16/CD32 antibody, BD Bioscience #101302) and stained for 30 minutes at 4°C with the antibodies.

The following antibodies were used:
anti-CD45-VioGreen (cod. 130-123-900), anti-CD3-FITC (cod. 130-119-135), anti-CD4-APC/Vio770 (cod. 130-119-134), anti-CD8-VioBlue (cod. 130-123-865), anti-CD49b-PE (cod. 130-102-337), anti-CD11b-FITC (cod. 130-113-234), anti-F4/80-PE/Vio770 (cod. 130-118-459), anti-Ly6C-APC/Vio770 (cod. 130-111-917), anti-Ly6G-VioBlue (cod. 130-119-986), anti-MHCII-APC (cod. 130-112-388), anti-CD11c APC (cod. 130-110-839) (tutti Miltenyi Biotec) and anti-CD206-PE (cod. 141706, Biolegend). The samples were acquired with BD FACS Celesta.

### FKBP12 silencing through shRNA

The viral vector AAV9-shFKBP12 was constructed to induce FKBP12 silencing specifically in muscle.

8 sequences of shFKBP12, available in the TRC shRNA database, were drawn and cloned inside the viral vector AAV CRE:gRNA. Correct cloning was verified by vector sequencing.

The sequences were subsequently validated for the actual silencing of the FKBP12 target. Of the 8 cloned sequences, only sequence #9 (TRCN0000012492) gave sufficient silencing of the FKBP12 target, combined with an increased expression of ID3 in C2C12 murine myotubes (Fig. 5d, e). Although the other sequences had been partially validated previously in the literature, they did not give a sufficient result in the context analyzed.

Therefore, 10¹¹ AAV9-shFKBP12#9 viral particles were injected in the left gastrocnemius of 3-month-old Balb-C mice, and 1011 AAV9-shScramble viral particles into the right contralateral, as an internal control.

After one month of injection, the mice were inoculated with colon-26 (C26) tumor cells to verify that the specific muscle silencing of FKBP12 was sufficient to protect the muscles from atrophy. 11 days after inoculation with C26, the mice were sacrificed and their muscles weighed. Both the gastrocnemius and quadriceps were found to be heavier than the respective shScramble control (Fig. 5f, g), and the FKBP12 target protein levels were actually decreased at the level of the gastrocnemius (Fig. 5h).

### Statistical analysis

All the graphs show the mean ± SEM. N represents the number of independent experiments. Statistical significance was measured with the two-tailed Student's t-test or with the one-way ANOVA with Tukey's post-hoc test. P < 0.05 was considered statistically significant.

### Example 1: Tacrolimus prevents myotube atrophy through the BMP-SMAD1/5/8 signal pathway

To characterize the molecular mechanism of tacrolimus on skeletal muscle *in vitro,* C2C12 murine myotubes were treated with Activin A (ACTA), colon-26 tumor cell (C26 CM) conditioned medium and BMP6, and their diameter was measured to establish the level of atrophy. Tacrolimus is not only capable of preventing ACTA-induced atrophy and C26 CM, but also induces levels of hypertrophy comparable to BMP6, used as a positive control for the BMP-SMAD1/5/8 signal pathway. (Fig.1 a-c) Unexpectedly, in fact, tacrolimus is capable of increasing the phosphorylation levels of SMAD1/5, without the stimulation of any extracellular ligand. (Fig.1 d-e) Furthermore, the transcriptional capacity of the SMAD1/5/8 proteins was measured with the pGL3-BMP (BRE)-Luc reporter vector, demonstrating how tacrolimus is even capable of exceeding the BMP6 positive control in reporter activation in C2C12 murine myoblasts. (Fig. 1f)

To further demonstrate the effect of tacrolimus on myotube mass, the protein ubiquitination flow was measured following proteasome inhibition. As expected, ACTA induces a significant increase in protein ubiquitination, completely reversed by treatment with tacrolimus (Fig. 1g).

Given the biological promiscuity of the FKBP12 tacrolimus target protein, the myotubes were treated with ciclosporin A (CyA), an FKBP12-independent calcineurin inhibitor. The diameter of the myotubes does not reflect the level of hypertrophy induced by tacrolimus, but instead induces an atrophy of about 20% (Fig. 1h). Accordingly, it is demonstrated that the biological effect of tacrolimus on myotubes should be independent of calcineurin inhibition.

To further demonstrate that FKBP12 is the biological target of tacrolimus, the C2C12 myotubes were silenced by the FKBP12 transcript with RNA interference technology (siFKBP12). Similar to treatment with tacrolimus, siFKBP12 is capable of protecting against C26 CM-induced atrophy (Fig. 1i-j).

### Example 2: Tacrolimus improves the conditions of murine models of tumor cachexia.

The Colon-26 (C26) murine tumor cachexia model (10) was used to test the *in vivo* efficacy of tacrolimus. This model develops a strong muscular atrophy, during the 11 days following the inoculation of the tumor cells of the murine adenocarcinoma colon-26. The mice were treated orally with daily doses of tacrolimus approximately 40 times less than the immunosuppressive doses.

The treatment is capable of preventing heavy weight and fat loss in C26 mice without altering the weight of the tumor. (Fig. 2a-c) Furthermore, the Grip Strength test demonstrates increased maximum muscle strength in C26 treated at the end of the experiment. (Fig. 2d) Consistently, tacrolimus is capable of preventing muscle atrophy, both in terms of skeletal muscle weight and in terms of muscle fiber diameter. (Fig. 2e-f) A further marker of atrophy protection is the reduced expression of all the atrogens in the gastrocnemii of the C26 mice treated with the drug. (Fig. 2g)

Finally, although the doses used were extremely lower than the immunosuppressive ones, to further exclude any possible alteration of the immune system, the amount and the state of activation of the main immune populations in the blood, spleens (data not shown) and tumors was measured, demonstrating that the treatment with tacrolimus has no effect on these parameters. (Fig. 3)

### Example 3: Tacrolimus and muscle hypertrophy

The treatment with FK506 is not only capable of preventing C26 CM-induced atrophy in C2C12 myotubes, but is also capable of inducing significant hypertrophy after 24h of treatment (Fig. 4a).

Similarly, FK506 is capable of increasing the mass of the quadriceps *in vivo,* in Balb C mice treated orally with non-immunosuppressive doses for 11 days (Fig. 4b).

Furthermore, the prolonged treatment with FK506 significantly increases maximum muscle strength with respect to vehicle-treated mice (CTR) at day 29 of treatment. (Fig. 4c)

### Example 4: silencing experiments

To further confirm the importance of the tacrolimus FKBP12 target, the BMP receptor which is found to be inhibited by this protein was identified. The silencing of two different BMP receptors, ALK2 and ALK3, identified ALK2 as the possible interactor of FKBP12, since its silencing nullifies the effect of tacrolimus in terms of diameter (Fig. 5a) and phosphorylation of the Smad1/5/8 proteins in C2C12 myotubes. (Fig. 5b) This data had never been provided at the muscular level, but it appears to be in line with the literature relating to the liver (4).

Further evidence of the dependence of Smad1/5/8 for the biological effect of tacrolimus is provided by the experiment with the Smad1/5/8 inhibitor, DMH1 (CAS No 1206711-16- 1). Such a treatment nullifies both the hypertrophic effect of tacrolimus and the protection of C26 (C26 CM) tumor medium-induced atrophy, further demonstrating the involvement of this pathway for the functioning of the drug. (Fig. 5c)

Therefore, the viral vector AAV9-shFKBP12 was constructed, to induce FKBP12 silencing specifically in muscle, to reproduce the protection obtained with tacrolimus.

8 sequences of shFKBP12, available in the TRC shRNA database, were drawn and cloned inside the viral vector AAV CRE:gRNA. Correct cloning was verified by vector sequencing.

The sequences were subsequently validated for the actual silencing of the FKBP12 target. Of the 8 cloned sequences, only sequence #9 (TRCN0000012492) gave sufficient silencing of the FKBP12 target, combined with increased expression of ID3 in C2C12 murine myotubes. (Fig. 5d, e). Although the other sequences had been partially validated previously in the literature, they did not give a sufficient result in the context analyzed.

Therefore, 10¹¹ AAV9-shFKBP12#9 viral particles were injected in the left gastrocnemius of 3-month-old Balb-C mice, and 1011 AAV9-shScramble viral particles into the right contralateral, as an internal control.

After one month of injection, the mice were inoculated with colon-26 (C26) tumor cells to verify that the specific muscle silencing of FKBP12 was sufficient to protect the muscles from atrophy. 11 days after inoculation with C26, the mice were sacrificed and their muscles weighed. Both the gastrocnemius and quadriceps were found to be heavier than the respective shScramble control (Fig. 5f, g), and the FKBP12 target protein levels were actually decreased at the gastrocnemius level, validating the hypothesized mechanism of action. (Fig. 5h)

### DISCUSSION

Despite having an incidence of 80% of cancer patients (1), neoplastic cachexia is still untreated. In this study, an "off-label" use of the drug tacrolimus (FK506) is proposed, at a dose lower than that used to obtain immunosuppression, for the treatment of muscle atrophy induced by tumor cachexia, acting on the molecular mechanism of the process.

Such a molecular mechanism has been characterized in vitro in C2C12 myotubes, where tacrolimus is capable of preventing C26 CM and ACTA-induced atrophy and inducing significant hypertrophy. Since tacrolimus is also a calcineurin inhibitor (CNI), and it is known that high doses of CNI can increase the incidence of solid tumors (11), unlike previous studies, low non-immunosuppressive doses have been used, so as not to interfere with the activation status of calcineurin and the immune system. (12) Further evidence of the independence of calcineurin from this pharmacological effect is the significant atrophy of the myotubes following treatment with ciclosporin A.

Once the role of calcineurin was excluded, the ability of tacrolimus to interfere with the BMP-SMAD1/5/8 axis was evaluated, based on recent studies on the disturbance of the interaction between ALK2 and FKBP12 in hepatocytes. (4) Similarly, some studies are demonstrating that non-immunosuppressive doses of tacrolimus can have beneficial effects in models of pulmonary hypertension (13) and chronic renal failure (14), due to its enhancement of the BMP-SMAD1/5/8 signal pathway. As hypothesized, signaling and luminescence experiments demonstrate an increased phosphorylation and transcriptional activity of SMAD 1/5/8 complexes following treatment with tacrolimus. Consistent with the positive effect of tacrolimus on the diameter of myotubes, it is the reduction of ubiquitinated protein flow which reflects reduced protein degradation in myotubes treated with tacrolimus with and without ACTA.

Furthermore, in the present model, the involvement of FKBP12 immunophilin is tested by RNA interference experiments or by shRNA, where FKBP12 silencing is able to prevent C26 CM-induced atrophy. As the silencing of FKBP12 does not fully reflect tacrolimus-induced hypertrophy, the involvement of other immunophilins of the FK506-binding proteins (FKBPs) family is not excluded.

Regarding the effects of tacrolimus in vivo, although known doses have been used to be non-immunosuppressive, the activation parameters and the quantity of the main immune populations have nevertheless been evaluated in order to exclude phenomena of immune tolerance.

In fact, if the role of the TGF_{β} superfamily on tumor cells was found to be strictly dependent on the type of tumor (15, 16), with regard to the immune system, it is established that the signaling of BMP has positive effects on tumor immunosurveillance (17-19), while the signaling of TGF_{β} suppresses the activation of such immune populations towards neoplastic tissue. (20, 21)

Despite speculation as to the possible effect of tacrolimus on the tumor microenvironment, the analyses performed demonstrate that the dose of tacrolimus used in vivo is not sufficient to interfere in any way with the immune populations, allowing lowdose "off-label" use for its interaction with BMP-SMAD and FKBP12 signaling. Therefore, the effect of the drug is limited to an increased maximum muscle mass and strength, as well as an absence of adipose tissue loss in C26 tumor mice treated with tacrolimus.

### REFERENCES

1. Argiles JM, Stemmler B, Lopez-Soriano FJ, Busquets S. Inter-tissue communication in cancer cachexia. Nat Rev Endocrinol. 2018;15(1):9-20.
2. Porporato PE. Understanding cachexia as a cancer metabolism syndrome. Oncogenesis. 2016;5:e200.
3. Sartori R, Gregorevic P, Sandri M. TGFbeta and BMP signaling in skeletal muscle: potential significance for muscle-related disease. Trends Endocrinol Metab. 2014;25(9):464-71.
4. Colucci S, Pagani A, Pettinato M, Artuso I, Nai A, Camaschella C, et al. The immunophilin FKBP12 inhibits hepcidin expression by binding the BMP type I receptor ALK2 in hepatocytes. Blood. 2017;130(19):2111-20.
5. Tamura K, Fujimura T, Iwasaki K, Sakuma S, Fujitsu T, Nakamura K, et al. Interaction of tacrolimus(FK506) and its metabolites with FKBP and calcineurin. Biochem Biophys Res Commun. 1994;202(1):437-43.
6. Tando T, Hirayama A, Furukawa M, Sato Y, Kobayashi T, Funayama A, et al. Smad2/3 Proteins Are Required for Immobilization-induced Skeletal Muscle Atrophy. J Biol Chem. 2016;291(23):12184-94.
7. Chen JL, Walton KL, Hagg A, Colgan TD, Johnson K, Qian H, et al. Specific targeting of TGF-beta family ligands demonstrates distinct roles in the regulation of muscle mass in health and disease. Proc Natl Acad Sci US A. 2017;114(26):E5266- E75.
8. Peiffer BJ, Qi L, Ahmadi AR, Wang Y, Guo Z, Peng H, et al. Activation of BMP Signaling by FKBP12 Ligands Synergizes with Inhibition of CXCR4 to Accelerate Wound Healing. Cell Chem Biol. 2019;26(5):652-61 e4.
9. Wyart E, Reano S, Hsu MY, Longo DL, Li M, Hirsch E, et al. Metabolic Alterations in a Slow-Paced Model of Pancreatic Cancer-Induced Wasting. Oxid Med Cell Longev. 2018;2018:6419805.
10. Bonetto A, Rupert JE, Barreto R, Zimmers TA. The Colon-26 Carcinoma Tumorbearing Mouse as a Model for the Study of Cancer Cachexia. J Vis Exp. 2016(117).
11. Carenco C, Assenat E, Faure S, Duny Y, Danan G, Bismuth M, et al. Tacrolimus and the risk of solid cancers after liver transplant: a dose effect relationship. Am J Transplant. 2015;15(3):678-86.
12. Shihab FS, Bennett WM, Tanner AM, Andoh TF. Mechanism of fibrosis in experimental tacrolimus nephrotoxicity. Transplantation. 1997;64(12):1829-37.
13. Spiekerkoetter E, Tian X, Cai J, Hopper RK, Sudheendra D, Li CG, et al. FK506 activates BMPR2, rescues endothelial dysfunction, and reverses pulmonary hypertension. J Clin Invest. 2013;123(8):3600-13.
14. Tampe B, Tampe D, Nyamsuren G, Klopper F, Rapp G, Kauffels A, et al. Pharmacological induction of hypoxia-inducible transcription factor ARNT attenuates chronic kidney failure. J Clin Invest. 2018;128(7):3053-70.
15. Scheel C, Eaton EN, Li SH, Chaffer CL, Reinhardt F, Kah KJ, et al. Paracrine and autocrine signals induce and maintain mesenchymal and stem cell states in the breast. Cell. 2011;145(6):926-40.
16. Jung B, Staudacher JJ, Beauchamp D. Transforming Growth Factor beta Superfamily Signaling in Development of Colorectal Cancer. Gastroenterology. 2017;152(1):36-52.
17. Martinez VG, Hidalgo L, Valencia J, Hernandez-Lopez C, Entrena A, del Amo BG, et al. Autocrine activation of canonical BMP signaling regulates PD-L1 and PD-L2 expression in human dendritic cells. Eur J Immunol. 2014;44(4):1031-8.
18. Gorelik L, Flavell RA. Immune-mediated eradication of tumors through the blockade of transforming growth factor-beta signaling in T cells. Nat Med. 2001;7(10):1118-22.
19. Kopp HG, Placke T, Salih HR. Platelet-derived transforming growth factor-beta down-regulates NKG2D thereby inhibiting natural killer cell antitumor reactivity. Cancer Res. 2009;69(19):7775-83.
20. Yang L, Pang Y, Moses HL. TGF-beta and immune cells: an important regulatory axis in the tumor microenvironment and progression. Trends Immunol. 2010;31(6):220-7.
21. Thomas DA, Massague J. TGF-beta directly targets cytotoxic T cell functions during tumor evasion of immune surveillance. Cancer Cell. 2005;8(5):369-80.
22. Sartori R, Schirwis E, Blaauw B, Bortolanza S, Zhao J, Enzo E et al. BMP signaling controls muscle mass. Nature genetics 2013; 45(11):1309-1318.
23. Craig A, Goodman and Troy A Hornberger. New roles for Smad signaling and phosphatidic acid in the regulation of skeletal muscle mass. F1000 Prime Reports 2014; 6:20
24. Chenlei Z, Wang C, Zhang T, Li D, Ni X, Lin JH et al. Exploring the Mechanism of Skeletal Muscle in a Tacrolimus-induced Posttransplantation Diabetes Mellitus Model on Gene Expression Profiles. J. Diabetes Res. 2020; 1-11.25.
25. Bradford STJ, Ranghini EJ, Grimley E, Lee PH, Dressler GR, High-throughput screens for agonists of bone morphogenetic protein (BMP) signaling identify potent benzoxazole compounds. J. Biol. Chem. 2019; 294(9):3125-3136
26. Lepparanta O, Tikkanen J M, Bespalov MM, Koli K, Myllarniemi M. Bone morphogenetic protein-inducer tilorone identified by high-throughput screening is antifibrotic in vivo. Am. J. of Resp. Cell and Mol. Biol. 2013; 48(4):448-455

## Claims

1. An activator of the BMP-SMAD signaling pathway for use in the treatment and/or prevention of muscle atrophy, wherein said activator is an inhibitor of FKBP12 not inhibiting mTOR selected from the group consisting of tacrolimus or an analogue thereof selected from FKVP, L-658,818 or V-10,367 or GPI-1046 or GPI-1485, FK523 or FK520, or 36,37-dihydro-FK506, used at doses which do not induce immunosuppression, siRNA targeting *FKBP12,* and shRNA targeting *FKBP12.*

2. The activator of the BMP-SMAD signaling pathway for use according to claim 1 wherein the FKBP12 inhibitor displaces FKBP12 from a BMP receptor, preferably said receptor is ALK1, ALK2 or ALK3.

3. The activator of the BMP-SMAD signaling pathway for use according to any one of the preceding claims wherein the muscle atrophy is selected from the group consisting of: neoplastic cachexia, bed rest or immobilization syndrome, atrophy associated with condition of heart and / or renal failure, atrophy associated with systemic inflammatory response syndrome (SIRS), preferably the systemic inflammatory response syndrome is AIDS, COPD, sepsis or hepatic steatosis.

4. The activator of the BMP-SMAD signaling pathway for use according to any one of the preceding claims wherein the muscle atrophy is **characterized by** a reduced BMP-SMAD axis and / or an increase in the signaling pathway ACTA/ TGF_{β}-SMAD2/3.

5. The activator of the BMP-SMAD signaling pathway for use according to any of the preceding claims wherein the inhibitor of FKBP12 is used in combination with agonists of the BMP signaling pathway.

6. An activator of the BMP-SMAD signaling pathway for use in a therapeutic method for promotion and/or induction of muscle hypertrophy in a patient in need thereof, wherein said activator is an inhibitor of FKBP12 not inhibiting mTOR selected from the group consisting of tacrolimus or an analogue thereof selected from FKVP, L-658,818 or V-10,367 or GPI-1046 or GPI-1485, FK523 or FK520, or 36,37-dihydro-FK506, used at doses which do not induce immunosuppression, siRNA targeting *FKBP12,* and shRNA targeting *FKBP12*

7. A pharmaceutical composition comprising an activator of the BMP-SMAD signaling pathway as defined in any one of claims 1 to 2 for use in the treatment and / or prevention of a muscle atrophy in a patient in need thereof, or for use in a therapeutic method for promoting and / or the induction of muscle hypertrophy in a patient in need thereof, preferably said composition further comprising a therapeutic agent.

8. The pharmaceutical composition according to claim 7 for use in the treatment and/or prevention of a muscle atrophy selected from the group consisting of: neoplastic cachexia, bed rest or immobilization syndrome, atrophy associated with condition of heart and / or renal failure, atrophy associated with systemic inflammatory response syndrome (SIRS), preferably the systemic inflammatory response syndrome is AIDS, COPD, sepsis or hepatic steatosis.

## Patentansprüche

1. Aktivator des BMP-SMAD-Signalwegs zur Verwendung bei der Behandlung und/oder Vorbeugung von Muskelatrophie, wobei der Aktivator ein Inhibitor von FKBP12 ist, der mTOR nicht hemmt, ausgewählt aus der Gruppe bestehend aus Tacrolimus oder einem Analogon davon, ausgewählt aus FKVP, L-658,818 oder V-10,367 oder GPI-1046 oder GPI-1485, FK523 oder FK520 oder 36,37-Dihydro-FK506, verwendet in Dosen, die keine Immunsuppression induzieren, siRNA, die auf *FKBP12* abzielt, und shRNA, die auf *FKBP12* abzielt.

2. Aktivator des BMP-SMAD-Signalwegs zur Verwendung nach Anspruch 1, wobei der FKBP12-Inhibitor FKBP12 von einem BMP-Rezeptor verdrängt, wobei der Rezeptor vorzugsweise ALK1, ALK2 oder ALK3 ist.

3. Aktivator des BMP-SMAD-Signalwegs zur Verwendung nach einem der vorstehenden Ansprüche, wobei die Muskelatrophie aus der Gruppe ausgewählt ist, die aus neoplastischer Kachexie, Bettruhe- oder Immobilisierungssyndrom, Atrophie im Zusammenhang mit einem Zustand des Herz- und/oder Nierenversagens, Atrophie im Zusammenhang mit einem systemischen Entzündungsreaktionssyndrom (SIRS) besteht, wobei das systemische Entzündungsreaktionssyndrom vorzugsweise AIDS, COPD, Sepsis oder Lebersteatose ist.

4. Aktivator des BMP-SMAD-Signalwegs zur Verwendung nach einem der vorstehenden Ansprüche, wobei die Muskelatrophie durch eine verminderte BMP-SMAD-Achse und/oder eine Erhöhung des Signalwegs ACTA/TGF_{β}-SMAD2/3 gekennzeichnet ist.

5. Aktivator des BMP-SMAD-Signalwegs zur Verwendung nach einem der vorstehenden Ansprüche, wobei der Inhibitor von FKBP12 in Kombination mit Agonisten des BMP-Signalwegs verwendet wird.

6. Aktivator des BMP-SMAD-Signalwegs zur Verwendung bei einem therapeutischen Verfahren zur Förderung und/oder Induktion von Muskelhypertrophie bei einem Patienten, der dies benötigt, wobei der Aktivator ein Inhibitor von FKBP12 ist, der mTOR nicht hemmt, ausgewählt aus der Gruppe bestehend aus Tacrolimus oder einem Analogon davon, ausgewählt aus FKVP, L-658,818 oder V-10,367 oder GPI-1046 oder GPI-1485, FK523 oder FK520 oder 36,37-Dihydro-FK506, verwendet in Dosen, die keine Immunsuppression induzieren, siRNA, die auf *FKBP12* abzielt, und *shRNA,* die auf *FKBP12* abzielt.

7. Pharmazeutische Zusammensetzung, umfassend einen Aktivator des BMP-SMAD-Signalwegs, wie in einem der Ansprüche 1 bis 2 definiert, zur Verwendung bei der Behandlung und/oder Vorbeugung einer Muskelatrophie bei einem Patienten, der dies benötigt, oder zur Verwendung bei einem therapeutischen Verfahren zur Förderung und/oder Induktion von Muskelhypertrophie bei einem Patienten, der dies benötigt, wobei die Zusammensetzung vorzugsweise ferner ein therapeutisches Mittel umfasst.

8. Pharmazeutische Zusammensetzung nach Anspruch 7 zur Verwendung bei der Behandlung und/oder Vorbeugung einer Muskelatrophie, ausgewählt aus der Gruppe bestehend aus: neoplastischer Kachexie, Bettruhe- oder Immobilisierungssyndrom, Atrophie im Zusammenhang mit einem Zustand des Herz- und/oder Nierenversagens, Atrophie im Zusammenhang mit einem systemischen Entzündungsreaktionssyndrom (SIRS), wobei das systemische Entzündungsreaktionssyndrom vorzugsweise AIDS, COPD, Sepsis oder Lebersteatose ist.

## Revendications

1. Activateur de la voie de signalisation BMP-SMAD destiné à être utilisé dans le traitement et/ou la prévention de l'atrophie musculaire, dans lequel ledit activateur est un inhibiteur de FKBP12 n'inhibant pas mTOR choisi dans le groupe constitué par le tacrolimus ou un analogue de celui-ci choisi parmi FKVP, L-658,818 ou V-10,367 ou GPI-1046 ou GPI-1485, FK523 ou FK520, ou 36,37-dihydro-FK506, utilisé à des doses qui n'induisent pas d'immunosuppression, un siARN ciblant *FKBP12* et un shARN ciblant *FKBP12.*

2. Activateur de la voie de signalisation BMP-SMAD destiné à être utilisé selon la revendication 1 dans lequel l'inhibiteur de FKBP12 déplace FKBP12 d'un récepteur BMP, de préférence ledit récepteur est ALK1, ALK2 ou ALK3.

3. Activateur de la voie de signalisation BMP-SMAD destiné à être utilisé selon l'une quelconque des revendications précédentes dans lequel l'atrophie musculaire est choisie dans le groupe constitué par : la cachexie néoplasique, le syndrome d'alitement ou d'immobilisation, l'atrophie associée à une pathologie cardiaque et/ou une insuffisance rénale, l'atrophie associée au syndrome de réponse inflammatoire systémique (SIRS), de préférence le syndrome de réponse inflammatoire systémique est le SIDA, la BPCO, la septicémie ou la stéatose hépatique.

4. Activateur de la voie de signalisation BMP-SMAD destiné à être utilisé selon l'une quelconque des revendications précédentes dans lequel l'atrophie musculaire est **caractérisée par** une réduction de l'axe BMP-SMAD et/ou une augmentation de la voie de signalisation ACTA/TGF_{β}-SMAD2/3.

5. Activateur de la voie de signalisation BMP-SMAD destiné à être utilisé selon l'une quelconque des revendications précédentes dans lequel l'inhibiteur de FKBP12 est utilisé en combinaison avec des antagonistes de la voie de signalisation BMP.

6. Activateur de la voie de signalisation BMP-SMAD destiné à être utilisé dans une méthode thérapeutique destinée à promouvoir et/ou induire l'hypertrophie musculaire chez un patient en ayant besoin, dans lequel ledit activateur est un inhibiteur de FKBP12 n'inhibant pas mTOR choisi dans le groupe constitué par le tacrolimus ou un analogue de celui-ci choisi parmi FKVP, L-658,818 ou V-10,367 ou GPI-1046 ou GPI-1485, FK523 ou FK520, ou 36,37-dihydro-FK506, utilisé à des doses qui n'induisent pas d'immunosuppression, un siARN ciblant *FKBP12* et un shARN ciblant *FKBP12.*

7. Composition pharmaceutique comprenant un activateur de la voie de signalisation BMP-SMAD tel que défini dans l'une quelconque des revendications 1 à 12 destinée à être utilisée dans le traitement et/ou la prévention d'une atrophie musculaire chez un patient en ayant besoin, ou destinée à être utilisée dans une méthode thérapeutique destinée à promouvoir et/ou induire l'hypertrophie musculaire chez un patient en ayant besoin, de préférence ladite composition comprenant en outre un agent thérapeutique.

8. Composition pharmaceutique selon la revendication 7 destinée à être utilisée dans le traitement et/ou la prévention d'une atrophie musculaire choisie dans le groupe constitué par : la cachexie néoplasique, le syndrome d'alitement ou d'immobilisation, l'atrophie associée à une pathologie cardiaque et/ou une insuffisance rénale, l'atrophie associée au syndrome de réponse inflammatoire systémique (SIRS), de préférence le syndrome de réponse inflammatoire systémique est le SIDA, la BPCO, la septicémie ou la stéatose hépatique.
